# EUROPEAN PATENT SPECIFICATION

(11) **EP 4 072 301 B1**
(45) Date of publication and mention of the grant of the patent: **06.11.2024**
(21) Application number: 20824342.8
(22) Date of filing: 02.12.2020
(51) Int. Cl.: A23D 7/005, A23D 7/01, A24B 13/00, A24B 15/16, A24B 15/30

(54) **A POUCH FOR ORAL USE COMPRISING A NANOEMULSION**
EIN MUNDBEUTEL ZUR ORALEN VERWENDUNG ENTHALTEND EINER NANOEMULSION
UNE POCHETTE À USAGE ORAL COMPRENANT UNE NANOÉMULSION

(30) Priority: 09.12.2019 US 201962945423 P
(43) Date of publication of application: 19.10.2022
(73) Proprietor: Nicoventures Trading Limited, London Greater London WC2R 3LA (GB)
(72) Inventor: HUTCHENS, Ronald K., London Greater London WC2R 3LA (GB); POOLE, Thomas H., London Greater London WC2R 3LA (GB); VON COSMOS, Nicolas, London Greater London WC2R 3LA (GB); GERARDI, Anthony Richard, London Greater London WC2R 3LA (GB); GRIMES, Chris J., London Greater London WC2R 3LA (GB); ALDERMAN, Steven Lee, London Greater London WC2R 3LA (GB); HOLTON, JR., Darrell Eugene, London Greater London WC2R 3LA (GB); KELLER, Christopher, Advance, North Carolina 27066 (US)
(74) Representative: Newcombe, Christopher David
(86) International application number: PCT/IB2020/061394
(87) International publication number: WO 2021/116834

(56) References cited:
- WO-A1-2004/000273
- WO-A1-2009/067734
- WO-A1-2016/144376
- WO-A1-2016/147186
- WO-A1-2018/029626
- WO-A1-2019/115778
- WO-A1-2019/135224
- WO-A1-2020/236798
- US-A1- 2015 231 070
- LIU ET AL: "Food-Grade Nanoemulsions: Preparation, Stability and Application in Encapsulation of Bioactive Compounds", MOLECULES, vol. 24, no. 23, 21 November 2019 (2019-11-21), pages 4242, XP055730119, DOI: 10.3390/molecules24234242

## Description

### FIELD OF THE DISCLOSURE

The present disclosure relates to products intended for human use. The products are configured for oral use and deliver substances such as active ingredients during use. Such products may include tobacco or a product derived from tobacco, or may be tobacco-free alternatives.

### BACKGROUND

Tobacco may be enjoyed in a so-called "smokeless" form. Particularly popular smokeless tobacco products are employed by inserting some form of processed tobacco or tobacco-containing formulation into the mouth of the user. Conventional formats for such smokeless tobacco products include moist snuff, snus, and chewing tobacco, which are typically formed almost entirely of particulate, granular, or shredded tobacco, and which are either portioned by the user or presented to the user in individual portions, such as in single-use pouches or sachets. Other traditional forms of smokeless products include compressed or agglomerated forms, such as plugs, tablets, or pellets. Alternative product formats, such as tobacco-containing gums and mixtures of tobacco with other plant materials, are also known. See for example, the types of smokeless tobacco formulations, ingredients, and processing methodologies set forth in US Pat. Nos. 1,376,586 to Schwartz; 4,513,756 to Pittman et al.; 4,528,993 to Sensabaugh, Jr. et al.; 4,624,269 to Story et al.; 4,991,599 to Tibbetts; 4,987,907 to Townsend; 5,092,352 to Sprinkle, III et al.; 5,387,416 to White et al.; 6,668,839 to Williams; 6,834,654 to Williams; 6,953,040 to Atchley et al.; 7,032,601 to Atchley et al.; and 7,694,686 to Atchley et al.; US Pat. Pub. Nos. 2004/0020503 to Williams; 2005/0115580 to Quinter et al.; 2006/0191548 to Strickland et al.; 2007/0062549 to Holton, Jr. et al.; 2007/0186941 to Holton, Jr. et al.; 2007/0186942 to Strickland et al.; 2008/0029110 to Dube et al.; 2008/0029116 to Robinson et al.; 2008/0173317 to Robinson et al.; 2008/0209586 to Neilsen et al.; 2009/0065013 to Essen et al.; and 2010/0282267 to Atchley, as well as WO2004/095959 to Arnarp et al.

Smokeless tobacco product configurations that combine tobacco material with various binders and fillers have been proposed more recently, with example product formats including lozenges, pastilles, gels, extruded forms, and the like. See, for example, the types of products described in US Patent App. Pub. Nos. 2008/0196730 to Engstrom et al.; 2008/0305216 to Crawford et al.; 2009/0293889 to Kumar et al.; 2010/0291245 to Gao et al; 2011/0139164 to Mua et al.; 2012/0037175 to Cantrell et al.; 2012/0055494 to Hunt et al.; 2012/0138073 to Cantrell et al.; 2012/0138074 to Cantrell et al.; 2013/0074855 to Holton, Jr.; 2013/0074856 to Holton, Jr.; 2013/0152953 to Mua et al.; 2013/0274296 to Jackson et al.; 2015/0068545 to Moldoveanu et al.; 2015/0101627 to Marshall et al.; and 2015/0230515 to Lampe et al.. WO2019135224 discloses taste-enhanced cannabinoid submicron emulsion syrup compositions; WO2009067734 discloses nanoemulsions; US2015231070 discloses composition for nanoemulsion delivery systems; WO2018029626 discloses chia oil edible nanoemulsions; WO2016147186 discloses preparations of cannabis emulsions and methods thereof; WO2016144376 discloses lipid nanoparticles compositions and methods as carrier of cannabinoids in standardized precision-metered dosage forms; Lui et al, Molecules, vol 24, no 23, 21 Nov 2019 discloses food grade nanoemulsions; WO2004000273 discloses oral pharmaceutical forms of liquid drugs having improved bioavailability; WO2020236798 discloses nanoemulsion compositions comprising biologically active ingredients; and WO2019115778 discloses a flavoured moist oral pouched nicotine product comprising monoglyceride.

All-white snus portions are growing in popularity, and offer a discrete and aesthetically pleasing alternative to traditional snus. Such modern "white" pouched products may include a bleached tobacco or may be tobacco-free.

### BRIEF SUMMARY

The present disclosure generally provides a pouched product configured for oral use, comprising a filler and a nanoemulsion dispersed in or disposed on the filler, the nanoemulsion comprising a vegetable or mineral oil; water; from 0.01% to 15% by weight of an emulsifying agent; and an active ingredient

The active ingredient is selected from the group consisting of botanical materials, stimulants, amino acids, vitamins, antioxidants, nicotine components, cannabinoids, cannabimimetics, terpenes, nutraceuticals, pharmaceutical agents, and combinations thereof.

The emulsifying agent is a surfactant, a phospholipid, an amphiphilic polysaccharide, an amphiphilic protein, or a combination thereof. In some embodiments, the emulsifying agent is an ionic or non-ionic surfactant. In some embodiments, the emulsifying agent comprises Tween 20, Tween 80, Span 20, Span 40, Span 60, Span 80, lecithin, a hydrocolloid gum, a modified starch, or a combination thereof.

In some embodiments, the nanoemulsion further comprises a stabilizer selected from the group consisting of polysaccharides and polyols.

The nanoemulsion comprises nanoparticles having a size of from about 20 to about 200 nm. In some embodiments, the zeta potential of the nanoparticles is from about -40 mV to about 40 mV. In some embodiments, the nanoemulsion comprises particles having a polydispersity index of less than about 0.3.

These and other features, aspects, and advantages of the disclosure will be apparent from a reading of the following detailed description together with the accompanying drawings, which are briefly described below.

### BRIEF DESCRIPTION OF THE DRAWING

Having thus described aspects of the disclosure in the foregoing general terms, reference will now be made to the accompanying drawing, which is not necessarily drawn to scale. The drawing is exemplary only, and should not be construed as limiting the disclosure.

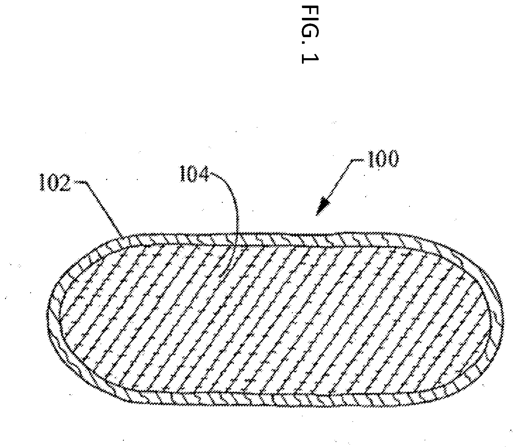

Fig. 1 is a perspective view of a pouched product embodiment, taken across the width of the product, showing an outer pouch filled with a composition of the present disclosure.

### DETAILED DESCRIPTION

The invention is defined as in the appended claims.

The present disclosure provides nanoemulsions including an oil, water, an active ingredient, and an emulsifying agent. Such nanoemulsions are configured for oral use, for example, in a composition enclosed within a pouch to form a pouched product.

The present disclosure will now be described more fully hereinafter with reference to example embodiments thereof. These example embodiments are described so that this disclosure will be thorough and complete, and will fully convey the scope of the disclosure to those skilled in the art. Indeed, the disclosure may be embodied in many different forms and should not be construed as limited to the embodiments set forth herein; rather, these embodiments are provided so that this disclosure will satisfy applicable legal requirements. As used in this specification and the claims, the singular forms "a," "an," and "the" include plural referents unless the context clearly dictates otherwise. Reference to "dry weight percent" or "dry weight basis" refers to weight on the basis of dry ingredients (i.e., all ingredients except water). Reference to "wet weight" refers to the weight of the composition including water. Unless otherwise indicated, reference to "weight percent" of a composition reflects the total wet weight of the composition (i.e., including water).

A nanoemulsion is a colloidal particulate system with particulates in the submicron size range. The particulates (referred to herein also as droplets or particles) are generally solid spheres, and the surfaces of such particulates are amorphous and lipophilic with a negative charge. Nanoemulsions generally comprise nano-scale particles having an average size of less than about 1,000 nm, for example, from about 10 to about 1,000 nm. Nanoemulsions as described herein comprise nanoparticles of oil emulsified in water and typically further comprise an emulsifying agent, an active ingredient, and/or a flavorant. The relative amounts of these various components within the nanoemulsion may vary, and typically are selected so as to provide the desired sensory and performance characteristics to the nanoemulsion. The example individual components of the nanoemulsion are described herein below.

### Oil

Any suitable oil may be used to form the nanoemulsion as disclosed herein, including petroleum-based (e.g., mineral oil) and natural or naturally derived oils (e.g., from plant materials or animal sources). In some embodiments, the oil is a food grade oil, including fractionated oils. Such oils include, but are not limited to, vegetable oils (e.g., acai oil, almond oil, amaranth oil, apricot oil, apple seed oil, argan oil, avocado oil, babassu oil, beech nut oil, ben oil, bitter gourd oil, black seed oil, blackcurrant seed oil, borage seed oil, borneo tallow nut oil, bottle gourd oil, brazil nut oil, buffalo gourd oil, butternut squash seed oil, cape chestnut oil, canola oil, carob cashew oil, cocoa butter, cocklebur oil, coconut oil, corn oil, cothune oil, coriander seed oil, cottonseed oil, date seed oil, dika oil, egus seed oil, evening primrose oil, false flax oil, flaxseed oil, grape seed oil, grapefruit seed oil, hazelnut oil, hemp oil, kapok seed oil, kenaf seed oil, lallemantia oil, lemon oil, linseed oil, macadamia oil, mafura oil, marula oil, meadowfoam seed oil, mongongo nut oil, mustard oil, niger seed oil, nutmeg butter, okra seed oil, olive oil, orange oil, palm oil, papaya seed oil, peanut oil, pecan oil, perilla seed oil, persimmon seed oil, pequi oil, pili nut oil, pine nut oil, pistachio oil, pomegranate seed oil, poppyseed oil, pracaxi oil, prune kernel oil, pumpkin seed oil, quinoa oil, ramtil oil, rapeseed oil, rice bran oil, royle oil, sacha inchi oil, safflower oil, sapote oil, seje oil, sesame oil, shea butter, soybean oil, sunflower oil, taramira oil, tea seed oil, thistle oil, tigernut oil, tobacco seed oil, tomato seed oil, walnut oil, watermelon seed oil, wheat germ oil, and combinations thereof), animal oils (e.g., cattle fat, buffalo fat, sheep fat, goat fat, pig fat, lard, camel fat, tallow, liquid margarine, fish oil, fish liver oil, whale oil, seal oil, and combinations thereof), and mineral oils.

In some embodiments, the oil comprises mineral oil. In some embodiments, the oil comprises a long chain fatty acid, a monoacylglycerol, a diacylglycerol, a triacylglycerol, or a combination thereof, wherein the acyl group is a long chain fatty acid. As used herein, "long chain fatty acid" refers to a carboxylic (CO₂H) acid having an aliphatic carbon chain of from about 11 to about 21 carbon atoms. The aliphatic carbon chain may be straight or branched. The aliphatic carbon chain may be saturated (i.e., having all *sp³* carbon atoms), or may be unsaturated (i.e., having at least one site of unsaturation). As used herein, the term "unsaturated" refers to the presence of a carbon-carbon, *sp²* double bond in one or more positions within the aliphatic carbon chain. Unsaturated alkyl groups may be mono- or polyunsaturated. Representative long chain fatty acids include, but are not limited to, undecylic acid, undecanoic acid, lauric acid, tridecanoic acid, myristic acid, pentadecanoic acid, palmitic acid, margaric acid, stearic acid, nonadecanoic acid, arachidic acid, heneicosanoic acid, α-linolenic acid, stearidonic acid, eicosapentaenoic acid, cervonic acid, linoleic acid, linolelaidic acid, γ-linolenic acid, dihomo-γ-linolenic acid, and arachidonic acid.

In some embodiments, the oil comprises an acyl glycerol, such as a monoacylglycerol, a diacylglycerol, or a triacylglycerol, wherein the acyl group is a long chain fatty acid as described herein. In some embodiments, the oil comprises polyunsaturated long chain fatty acids, or mono-di- or triacylglycerol containing polyunsaturated long chain fatty acids as the acyl component. The chain lengths of the fatty acids in naturally occurring triglycerides may vary, but is typically 16, 18, or 20 carbon atoms. In some embodiments, the concentration of polyunsaturated fatty acid (as free fatty acid or as e.g., triglycerides) in the oil can range from about 2% to 100% (w/w), such as from about 5% to 100% (w/w) or greater than 10%, e.g., 20%-80% (w/w).

In some embodiments, the oil comprises castor oil, corn oil, coconut oil, cod liver oil, evening primrose oil, cottonseed oil, palm oil, rice bran oil, sesame oil, rapeseed oil, canola oil, cocoa butter, linseed oil, olive oil, peanut oil, soybean oil, safflower oil, flaxseed oil, sunflower oil, olive oil, or a combination thereof.

The amount of oil present within the disclosed nanoemulsion can vary, but is typically from about 5% to about 80% by weight, or from about 10% to about 60% by weight, or from about 20% to about 50% by weight, based on the total weight of the nanoemulsion.

### Water

Nanoemulsions as disclosed herein comprise water. Water may be present as, for example, purified or ultrapure water, saline, buffered saline, or a buffered aqueous phase. The water content of the nanoemulsion may vary according to the desired properties. Typically, the water content will be from about 20 to about 90% by weight, based on the total weight of the nanoemulsion. In some embodiments, a further hydrophilic, water soluble component may be added to the water, including short chain mono-, di-, and polyhydric alcohols, (e.g., ethanol, benzyl alcohol, glycerol, propylene glycol, propylene carbonate, polyethylene glycol with an average molecular weight of about 200 to about 10,000, diethylene glycol monoethyl ether, and combinations thereof).

### Emulsifying agent

Nanoemulsions as disclosed herein comprise one or more emulsifying agents. By "emulsifying agent" is meant a substance which aids in the formation and stabilization of emulsions by promoting dispersion of hydrophobic and hydrophilic (e.g., oil and water) components. In general, emulsifiers are amphiphilic molecules chosen from, for example, nonionic and ionic amphiphilic molecules. The expression "amphiphilic molecule" means any molecule of bipolar structure comprising at least one hydrophobic portion and at least one hydrophilic portion and having the property of reducing the surface tension of water and of reducing the interface tension between water and an oily phase. Emulsifying agents/amphiphilic molecules as provided herein are also referred to as, for example, surfactants and emulsifiers.

In some embodiments, the emulsifying agent comprises neutral, positively charged, or negatively charged natural or synthetic phospholipids molecules. Phospholipids are made up of two fatty acid tails and a phosphate group head, connected via a third molecule, glycerol. Non-limiting examples of natural phospholipids including soybean lecithin, egg lecithin, phosphatidylglycerol, phosphatidylinositol, phosphatidylethanolamine, phosphatidic acid, sphingomyelin, diphosphatidylglycerol, phosphatidylserine, phosphatidylcholine and cardiolipin; synthetic phospholipids including dimyristoylphosphatidylcholine, dimyristoylphosphatidylglycerol, distearoylphosphatidylglycerol and dipalmitoylphosphatidylcholine; and hydrogenated or partially hydrogenated lecithins and phospholipids. Non-limiting examples of synthetic phospholipid derivatives include phosphatidic acid (DMPA, DPPA, DSPA), phosphatidylcholine (DDPC, DLPC, DMPC, DPPC, DSPC, DOPC, POPC, DEPC), phosphatidylglycerol (DMPG, DPPG, DSPG, POPG), phosphatidylethanolamine (DMPE, DPPE, DSPE DOPE), phosphatidylserine (DOPS), PEG phospholipid (mPEG-phospholipid, polyglycerin-phospholipid, functionalized-phospholipid, and terminal activated-phospholipid).

In some embodiments, the emulsifying agent comprises a surfactant, which may be ionic or non-ionic, and which may be hydrophobic or hydrophilic. Examples of hydrophobic surfactants include, but are not limited to, Maisine 35-1, Imwitor 742, Capmul MCM, Capmul PG 12, Lauroglycol 90, Lauroglycol FCC, Caproyl 90, Captex 250, a fatty acid selected from the group consisting of octanoic acid, decanoic acid, undecanoic acid, lauric acid, myristic acid, palmitic acid, stearic acid, oleic acid, linoleic acid, and linolenic acid. As used herein, a hydrophobic surfactant may also be referred to as a poorly water soluble surfactant or a lipophilic surfactant.

Examples of hydrophilic surfactants may include, but are not limited to polyoxyethylene sorbitan fatty acid esters, hydrogenated castor oil ethoxylates, PEG mono- and di-esters of palmitic and stearic acids, fatty acid ethoxylates, and combinations thereof.

Examples of suitable surfactants generally include, but are not limited to: polyoxyethylene-sorbitan-fatty acid esters; e.g., mono- and tri-lauryl, palmityl, stearyl and oleyl esters; e.g., products of the type known as polysorbates and commercially available under the trade name Tween^{®}; polyoxyethylene fatty acid esters, e.g., polyoxyethylene stearic acid esters of the type known and commercially available under the trade name Myrj^{®}; polyoxyethylene castor oil derivatives, e.g., products of the type known and commercially available as Cremophors^{®}. Particularly suitable are polyoxyl 35 castor oil (Cremophor^{®}EL) and polyoxyl 40 hydrogenated castor oil (Cremophor^{®}RH40); a- tocopherol, a-tocopheryl polyethylene glycol succinate (vitamin E TPGS), a- tocopherol palmitate and a-tocopherol acetate; PEG glyceryl fatty acid esters such as PEG-8 glyceryl caprylate/caprate (commercially known as Labrasol^{®}), PEG-4 glyceryl caprylate/caprate (Labrafac Hydro WL 1219), PEG-32 glyceryl laurate (Gelucire 44/14), PEG-6 glyceryl mono oleate (Labrafil^{®} M 1944 CS), PEG-6 glyceryl linoleate (Labrafil^{®} M 2125 CS); propylene glycol mono- and di-fatty acid esters, such as propylene glycol laurate, propylene glycol caprylate/caprate; also diethyleneglycol-monoethylether (DGME), commercially known as Transcutol^{®} (Gattefosse, Westwood, N.J.); sorbitan fatty acid esters, such as the type known and commercially available under the name Span^{®} (e.g., Span 85); polyoxyethylene-polyoxypropylene co-polymers, e.g., products of the type known and commercially available as Pluronic^{®} or Poloxamer^{®}; glycerol triacetate; and monoglycerides and acetylated monoglycerides, e.g., glycerol monodicocoate (Imwitor^{®} 928), glycerol monocaprylate (Imwitor^{®} 308), and mono-and di-acetylated monoglycerides.

The emulsifying agent is a surfactant, a phospholipid, an amphiphilic polysaccharide, an amphiphilic protein, or a combination thereof. In some embodiments, the emulsifying agent is an ionic or non-ionic surfactant. In some embodiments, the emulsifying agent comprises Tween 20, Tween 80, Span 20, Span 40, Span 60, Span 80, lecithin, a hydrocolloid gum, a modified starch, or a combination thereof.

The concentration of the emulsifying agent present in the disclosed nanoemulsion may vary. The concentration of the emulsifying agent is from about 0.01% to about 15%, optionally from about 0.1% to about 10%, or optionally from about 1% to about 5% by weight based on the entirety of the nanoemulsion.

### Stabilizer

In some embodiments, the nanoemulsion may further comprise a stabilizer to assist in maintaining the nanoemulsion. Representative examples of suitable types of stabilizers include polysaccharides, polyols, sorbitan esters, glycerol esters, polyethylene glycol esters, block polymers, acrylic polymers (such as Pemulen), silicon based surfactants, and polysorbates. In some embodiments, the stabilizer is sodium oleate, glycerine, xylitol, sorbitol, ascorbic acid, sodium edetate, a sorbitan ester, a glycerol monoester, or a combination thereof.

The concentration of the stabilizer present in the nanoemulsion may vary. When present, the concentration of the emulsifying agent may be in a range of up to about 10% by weight, for example from about 0.01% to about 10%, from about 0.1% to about 5%, or from about 0.5% to about 1% by weight based on the weight of the nanoemulsion.

### Active ingredient

The nanoemulsion as disclosed herein includes one or more active ingredients. In some embodiments, two or more active ingredients can be incorporated within the same nanoemulsion. As used herein, an "active ingredient" refers to one or more substances belonging to any of the following categories: API (active pharmaceutical substances), food additives, natural medicaments, and naturally occurring substances that can have an effect on humans. Example active ingredients include any ingredient known to impact one or more biological functions within the body, such as ingredients that furnish pharmacological activity or other direct effect in the diagnosis, cure, mitigation, treatment, or prevention of disease, or which affect the structure or any function of the body of humans (e.g., provide a stimulating action on the central nervous system, have an energizing effect, an antipyretic or analgesic action, or an otherwise useful effect on the body). In some embodiments, the active ingredient may be of the type generally referred to as dietary supplements, nutraceuticals, "phytochemicals" or "functional foods". These types of additives are sometimes defined in the art as encompassing substances typically available from naturally-occurring sources (e.g., botanical materials) that provide one or more advantageous biological effects (e.g., health promotion, disease prevention, or other medicinal properties), but are not classified or regulated as drugs.

Non-limiting examples of active ingredients include those falling in the categories of botanical ingredients (e.g., hemp, lavender, peppermint, eucalyptus, rooibos, fennel, cloves, chamomile, basil, rosemary, clove, citrus, ginger, cannabis, ginseng, maca, and tisanes), stimulants (e.g., caffeine or guarana), amino acids (e.g., taurine, theanine, phenylalanine, tyrosine, and tryptophan), vitamins (B6, B12, and C), antioxidants, nicotine components, pharmaceutical ingredients (e.g., nutraceutical and medicinal ingredients), cannabinoids (e.g., tetrahydrocannabinol (THC) or cannabidiol (CBD)) and/or melatonin.. Each of these categories is further described herein below. The particular choice of active ingredients will vary depending upon the desired flavor, texture, and desired characteristics of the particular product.

The particular percentages of active ingredients present within the disclosed emulsion will vary depending upon the desired flavor, texture, and other characteristics of the nanoemulsion and any product into which the nanoemulsion is incorporated. Typically, an active ingredient or combination thereof is present in a concentration of at least about 0.001% by weight of the nanoemulsion, such as in a range from about 0.001% to about 20%. In some embodiments, the active ingredient is present in a concentration from about 0.1% w/w to about 20% by weight, such as, e.g., from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20% by weight, based on the total weight of the nanoemulsion. In some embodiments, the active ingredient is present in a concentration from about 0.1% w/w to about 10% by weight, such as, e.g., from about 0.5% w/w to about 10%, from about 1% to about 10%, or about 1% to about 5% by weight, based on the total weight of the nanoemulsion.

### Botanical

In some embodiments, the active ingredient comprises a botanical ingredient. As used herein, the term "botanical ingredient" or "botanical" refers to any plant material or fungal-derived material, including plant material in its natural form and plant material derived from natural plant materials, such as extracts or isolates from plant materials or treated plant materials (e.g., plant materials subjected to heat treatment, fermentation, bleaching, or other treatment processes capable of altering the physical and/or chemical nature of the material). For the purposes of the present disclosure, a "botanical" includes, but is not limited to, "herbal materials," which refer to seed-producing plants that do not develop persistent woody tissue and are often valued for their medicinal or sensory characteristics (e.g., teas or tisanes). Reference to botanical material as "non-tobacco" is intended to exclude tobacco materials (i.e., does not include any *Nicotiana* species).

When present, a botanical is typically at a concentration of from about 0.01% w/w to about 10% by weight, such as, e.g., from about 0.01% w/w, about 0.05%, about 0.1%, or about 0.5%, to about 1%, about 2%, about 3%, about 4%, about 5%, about 6%, about 7%, about 8%, about 9%, or about 10%, about 11%, about 12%, about 13%, about 14%, or about 15% by weight, based on the total weight of the composition.

The botanical materials useful in the present disclosure may comprise, without limitation, any of the compounds and sources set forth herein, including mixtures thereof. Certain botanical materials of this type are sometimes referred to as dietary supplements, nutraceuticals, "phytochemicals" or "functional foods." Certain botanicals, as the plant material or an extract thereof, have found use in traditional herbal medicine, and are described further herein. Non-limiting examples of botanicals or botanical-derived materials include hemp, eucalyptus, rooibos, fennel, citrus, cloves, lavender, peppermint, chamomile, basil, rosemary, ginger, turmeric, green tea, white mulberry, cannabis, cocoa, ashwagandha, baobab, chlorophyll, cordyceps, damiana, ginseng, guarana, and maca. In some embodiments, the composition comprises green tea, turmeric, and white mulberry.

Ashwagandha (*Withania somnifera*) is a plant in the *Solanaceae* (nightshade) family. As an herb, Ashwagandha has found use in the Indian Ayurvedic system of medicine, where it is also known as "Indian Winter cherry" or "Indian Ginseng." In some embodiments, the active ingredient comprises ashwagandha.

Baobab is the common name of a family of deciduous trees of the genus *Adansonia.* The fruit pulp and seeds of the Baobab are consumed, generally after drying, as a food or nutritional supplement. In some embodiments, the active ingredient comprises baobab.

Chlorophyll is any of several related green pigments found in the mesosomes of cyanobacteria, as well as in the chloroplasts of algae and plants. Chlorophyll has been used as a food additive (colorant) and a nutritional supplement. Chlorophyll may be provided either from native plant materials (e.g., botanicals) or in an extract or dried powder form. In some embodiments, the active ingredient comprises chlorophyll.

Cordyceps is a diverse genus of ascomycete (sac) fungi which are abundant in humid temperate and tropical forests. Members of the cordyceps family are used extensively in traditional Chinese medicine. In some embodiments, the active ingredient comprises cordyceps.

Damiana is a small, woody shrub of the family *Passifloraceae.* It is native to southern Texas, Central America, Mexico, South America, and the Caribbean. Damiana produces small, aromatic flowers, followed by fruits that taste similar to figs. The extract from damiana has been found to suppress aromatase activity, including the isolated compounds pinocembrin and acacetin. In some embodiments, the active ingredient comprises damiana.

Guarana is a climbing plant in the family *Sapindaceae,* native to the Amazon basin. The seeds from its fruit, which are about the size of a coffee bean, have a high concentration of caffeine and, consequently, stimulant activity. In some embodiments, the active ingredient comprises guarana. In some embodiments, the active ingredient comprises guarana, honey, and ashwagandha.

Ginseng is the root of plants of the genus *Panax,* which are characterized by the presence of unique steroid saponin phytochemicals (ginsenosides) and gintonin. Ginseng finds use as a dietary supplement in energy drinks or herbal teas, and in traditional medicine. Cultivated species include Korean ginseng (*P*. *ginseng*), South China ginseng (*P. notoginseng*), and American ginseng (*P. quinquefolius*)*.* American ginseng and Korean ginseng vary in the type and quantity of various ginsenosides present. In some embodiments, the active ingredient comprises ginseng. In some embodiments, the ginseng is American ginseng or Korean ginseng. In specific embodiments, the active ingredient comprises Korean ginseng.

Maca is a plant that grows in central Peru in the high plateaus of the Andes Mountains. It is a relative of the radish, and has an odor similar to butterscotch. Maca has been used in traditional (e.g., Chinese) medicine. In some embodiments, the active ingredient comprises maca.

### Stimulants

In some embodiments, the active ingredient comprises one or more stimulants. As used herein, the term "stimulant" refers to a material that increases activity of the central nervous system and/or the body, for example, enhancing focus, cognition, vigor, mood, alertness, and the like. Non-limiting examples of stimulants include caffeine, theacrine, theobromine, and theophylline. Theacrine (1,3,7,9-tetramethyluric acid) is a purine alkaloid which is structurally related to caffeine, and possesses stimulant, analgesic, and anti-inflammatory effects. Present stimulants may be natural, naturally derived, or wholly synthetic. For example, certain botanical materials (guarana, tea, coffee, cocoa, and the like) may possess a stimulant effect by virtue of the presence of e.g., caffeine or related alkaloids, and accordingly are "natural" stimulants. By "naturally derived" is meant the stimulant (e.g., caffeine, theacrine) is in a purified form, outside its natural (e.g., botanical) matrix. For example, caffeine can be obtained by extraction and purification from botanical sources (e.g., tea). By "wholly synthetic", it is meant that the stimulant has been obtained by chemical synthesis.

When present, a stimulant or combination of stimulants (e.g., caffeine, theacrine, and combinations thereof) is typically at a concentration of from about 0.1% w/w to about 15% by weight, such as, e.g., from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about 13%, about 14%, or about 15% by weight, based on the total weight of the composition.

In some embodiments, the active ingredient comprises caffeine. In some embodiments, the active ingredient comprises theacrine. In some embodiments, the active ingredient comprises a combination of caffeine and theacrine.

### Amino acids

In some embodiments, the active ingredient comprises an amino acid. As used herein, the term "amino acid" refers to an organic compound that contains amine (-NH₂) and carboxyl (-COOH) or sulfonic acid (SOsH) functional groups, along with a side chain (R group), which is specific to each amino acid. Amino acids may be proteinogenic or non-proteinogenic. By "proteinogenic" is meant that the amino acid is one of the twenty naturally occurring amino acids found in proteins. The proteinogenic amino acids include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine. By "non-proteinogenic" is meant that either the amino acid is not found naturally in protein, or is not directly produced by cellular machinery (e.g., is the product of post-tranlational modification). Non-limiting examples of non-proteinogenic amino acids include gamma-aminobutyric acid (GABA), taurine (2-aminoethanesulfonic acid), theanine (L-γ-glutamylethylamide), hydroxyproline, and beta-alanine.

When present, an amino acid or combination of amino acids (e.g., taurine, theanine, and combinations thereof) is typically at a concentration of from about 0.1% w/w to about 15% by weight, such as, e.g., from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about 13%, about 14%, or about 15% by weight, based on the total weight of the composition.

In some embodiments, the amino acid is taurine, theanine, phenylalanine, tyrosine, tryptophan, or a combination thereof. In some embodiments, the amino acid is taurine. In some embodiments, the active ingredient comprises a combination of taurine and caffeine. In some embodiments, the active ingredient comprises a combination of taurine, caffeine, and guarana. In some embodiments, the active ingredient comprises a combination of taurine, maca, and cordyceps. In some embodiments, the active ingredient comprises a combination of theanine and caffeine.

### Vitamins

In some embodiments, the active ingredient comprises a vitamin or combination of vitamins. As used herein, the term "vitamin" refers to an organic molecule (or related set of molecules) that is an essential micronutrient needed for the proper functioning of metabolism in a mammal. There are thirteen vitamins required by human metabolism, which are: vitamin A (as all-trans-retinol, all-trans-retinyl-esters, as well as all-trans-beta-carotene and other provitamin A carotenoids), vitamin B1 (thiamine), vitamin B2 (riboflavin), vitamin B3 (niacin), vitamin B5 (pantothenic acid), vitamin B6 (pyridoxine), vitamin B7 (biotin), vitamin B9 (folic acid or folate), vitamin B12 (cobalamins), vitamin C (ascorbic acid), vitamin D (calciferols), vitamin E (tocopherols and tocotrienols), and vitamin K (quinones).

When present, a vitamin or combination of vitamins (e.g., vitamin B6, vitamin B12, vitamin E, vitamin C, or a combination thereof) is typically at a concentration of from about 0.01% w/w to about 1% by weight, such as, e.g., from about 0.01%, about 0.02%, about 0.03%, about 0.04%, about 0.05%, about 0.06%, about 0.07%, about 0.08%, about 0.09%, or about 0.1% w/w, to about 0.2%, about 0.3%, about 0.4%, about 0.5% about 0.6%, about 0.7%, about 0.8%, about 0.9%, or about 1% by weight, based on the total weight of the composition.

In some embodiments, the vitamin is vitamin B6, vitamin B12, vitamin E, vitamin C, or a combination thereof. In some embodiments, the active ingredient comprises a combination of vitamin B6, caffeine, and theanine. In some embodiments, the active ingredient comprises vitamin B6, vitamin B12, and taurine. In some embodiments, the active ingredient comprises a combination of vitamin B6, vitamin B12, ginseng, and theanine. In some embodiments, the active ingredient comprises a combination of vitamin C, baobab, and chlorophyll.

### Antioxidants

In some embodiments, the active ingredient comprises one or more antioxidants. As used herein, the term "antioxidant" refers to a substance which prevents or suppresses oxidation by terminating free radical reactions, and may delay or prevent some types of cellular damage. Antioxidants may be naturally occurring or synthetic. Naturally occurring antioxidants include those found in foods and botanical materials. Non-limiting examples of antioxidants include certain botanical materials, vitamins, polyphenols, and phenol derivatives.

Examples of botanical materials which are associated with antioxidant characteristics include without limitation acai berry, alfalfa, allspice, annatto seed, apricot oil, basil, bee balm, wild bergamot, black pepper, blueberries, borage seed oil, bugleweed, cacao, calamus root, catnip, catuaba, cayenne pepper, chaga mushroom, chervil, cinnamon, dark chocolate, potato peel, grape seed, ginseng, gingko biloba, Saint John's Wort, saw palmetto, green tea, black tea, black cohosh, cayenne, chamomile, cloves, cocoa powder, cranberry, dandelion, grapefruit, honeybush, echinacea, garlic, evening primrose, feverfew, ginger, goldenseal, hawthorn, hibiscus flower, jiaogulan, kava, lavender, licorice, marjoram, milk thistle, mints (menthe), oolong tea, beet root, orange, oregano, papaya, pennyroyal, peppermint, red clover, rooibos (red or green), rosehip, rosemary, sage, clary sage, savory, spearmint, spirulina, slippery elm bark, sorghum bran hi-tannin, sorghum grain hi-tannin, sumac bran, comfrey leaf and root, goji berries, gutu kola, thyme, turmeric, uva ursi, valerian, wild yam root, wintergreen, yacon root, yellow dock, yerba mate, yerba santa, bacopa monniera, withania somnifera, Lion's mane, and silybum marianum. Such botanical materials may be provided in fresh or dry form, essential oils, or may be in the form of an extracts. The botanical materials (as well as their extracts) often include compounds from various classes known to provide antioxidant effects, such as minerals, vitamins, isoflavones, phytoesterols, allyl sulfides, dithiolthiones, isothiocyanates, indoles, lignans, flavonoids, polyphenols, and carotenoids. Examples of compounds found in botanical extracts or oils include ascorbic acid, peanut endocarb, resveratrol, sulforaphane, beta-carotene, lycopene, lutein, co-enzyme Q, carnitine, quercetin, kaempferol, and the like. See, e.g., Santhosh et al., Phytomedicine, 12(2005) 216-220.

Non-limiting examples of other suitable antioxidants include citric acid, Vitamin E or a derivative thereof, a tocopherol, epicatechol, epigallocatechol, epigallocatechol gallate, erythorbic acid, sodium erythorbate, 4-hexylresorcinol, theaflavin, theaflavin monogallate A or B, theaflavin digallate, phenolic acids, glycosides, quercitrin, isoquercitrin, hyperoside, polyphenols, catechols, resveratrols, oleuropein, butylated hydroxyanisole (BHA), butylated hydroxytoluene (BHT), tertiary butylhydroquinone (TBHQ), and combinations thereof. In some embodiments, the antioxidant is Vitamin E or a derivative thereof, a flavonoid, a polyphenol, a carotenoid, or a combination thereof.

When present, an antioxidant is typically at a concentration of from about 0.001% w/w to about 10% by weight, such as, e.g., from about 0.001%, about 0.005%, about 0.01% w/w, about 0.05%, about 0.1%, or about 0.5%, to about 1%, about 2%, about 3%, about 4%, about 5%, about 6%, about 7%, about 8%, about 9%, or about 10%, based on the total weight of the composition.

### Cannabinoids

In some embodiments, the active ingredient comprises one or more cannabinoids. As used herein, the term "cannabinoid" refers to a class of diverse chemical compounds that acts on cannabinoid receptors, also known as the endocannabinoid system, in cells that alter neurotransmitter release in the brain. Ligands for these receptor proteins include the endocannabinoids produced naturally in the body by animals; phytocannabinoids, found in cannabis; and synthetic cannabinoids, manufactured artificially. Cannabinoids found in cannabis include, without limitation: cannabigerol (CBG), cannabichromene (CBC), cannabidiol (CBD), tetrahydrocannabinol (THC), cannabinol (CBN), cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), cannabinol propyl variant (CBNV), cannabitriol (CBO), tetrahydrocannabinolic acid (THCA), and tetrahydrocannabivarinic acid (THCV A). In certain embodiments, the cannabinoid is selected from tetrahydrocannabinol (THC), the primary psychoactive compound in cannabis, and cannabidiol (CBD) another major constituent of the plant, but which is devoid of psychoactivity. All of the above compounds can be used in the form of an isolate from plant material or synthetically derived.

Alternatively, the active ingredient can be a cannabimimetic, which is a class of compounds derived from plants other than cannabis that have biological effects on the endocannabinoid system similar to cannabinoids. Examples include yangonin, alpha-amyrin or beta-amyrin (also classified as terpenes), cyanidin, curcumin (tumeric), catechin, quercetin, salvinorin A, N-acylethanolamines, and N-alkylamide lipids.

When present, a cannabinoid (e.g., CBD) or cannabimimetic is typically in a concentration of at least about 0.1% by weight of the composition, such as in a range from about 0.1% to about 30%, such as, e.g., from about 0.1%, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 15%, about 20%, or about 30% by weight, based on the total weight of the composition.

### Terpenes

Active ingredients suitable for use in the present disclosure can also be classified as terpenes, many of which are associated with biological effects, such as calming effects. Terpenes are understood to have the general formula of (C₅H₈)ₙ and include monoterpenes, sesquiterpenes, and diterpenes. Terpenes can be acyclic, monocyclic or bicyclic in structure. Some terpenes provide an entourage effect when used in combination with cannabinoids or cannabimimetics. Examples include beta-caryophyllene, linalool, limonene, beta-citronellol, linalyl acetate, pinene (alpha or beta), geraniol, carvone, eucalyptol, menthone, iso-menthone, piperitone, myrcene, beta-bourbonene, and germacrene, which may be used singly or in combination.

### Pharmaceutical ingredients

The pharmaceutical ingredient can be any known agent adapted for therapeutic, prophylactic, or diagnostic use. These can include, for example, synthetic organic compounds, proteins and peptides, polysaccharides and other sugars, lipids, inorganic compounds, and nucleic acid sequences, having therapeutic, prophylactic, or diagnostic activity. Non-limiting examples of pharmaceutical ingredients include analgesics and antipyretics (e.g., acetylsalicylic acid, acetaminophen, 3-(4-isobutylphenyl)propanoic acid).

### Nicotine Component

In certain embodiments, a nicotine component may be included in the nanoemulsion. By "nicotine component" is meant any suitable form of nicotine (e.g., free base or salt) for providing oral absorption of at least a portion of the nicotine present. Typically, the nicotine component is selected from the group consisting of nicotine free base and a nicotine salt. In some embodiments, nicotine is in its free base form, which easily can be adsorbed in for example, a microcrystalline cellulose material to form a microcrystalline cellulose-nicotine carrier complex. See, for example, the discussion of nicotine in free base form in US Pat. Pub. No. 2004/0191322 to Hansson. As such, in some embodiments, a nanoemulsion is provided comprising a MCC-nicotine carrier complex.

In some embodiments, at least a portion of the nicotine can be employed in the form of a salt. Salts of nicotine can be provided using the types of ingredients and techniques set forth in U.S. Pat. No. 2,033,909 to Cox et al. and Perfetti, Beitrage Tabakforschung Int., 12: 43-54 (1983). Further salts are disclosed in, for example, U.S. Pat. No. 9,738,622 to Dull et al., and US Pat. Pub. Nos. 2018/0230126 to Dull et al., 2016/0185750 to Dull et al., and 2018/0051002 to Dull et al.. Additionally, salts of nicotine are available from sources such as Pfaltz and Bauer, Inc. and K&K Laboratories, Division of ICN Biochemicals, Inc. Typically, the nicotine component is selected from the group consisting of nicotine free base, a nicotine salt such as hydrochloride, dihydrochloride, monotartrate, bitartrate, sulfate, salicylate, and nicotine zinc chloride.

In some embodiments, at least a portion of the nicotine can be in the form of a resin complex of nicotine, where nicotine is bound in an ion-exchange resin, such as nicotine polacrilex, which is nicotine bound to, for example, a polymethacrilic acid, such as Amberlite IRP64, Purolite C115HMR, or Doshion P551. See, for example, US Pat. No. 3,901,248 to Lichtneckert et al.. Another example is a nicotine-polyacrylic carbomer complex, such as with Carbopol 974P. In some embodiments, nicotine may be present in the form of a nicotine polyacrylic complex.

Typically, the nicotine component (calculated as the free base) when present, is in a concentration of at least about 0.001% by weight of the nanoemulsion, such as in a range from about 0.001% to about 10%. In some embodiments, the nicotine component is present in a concentration from about 0.1% w/w to about 10% by weight, such as, e.g., from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, or about 10% by weight, calculated as the free base and based on the total weight of the nanoemulsion. In some embodiments, the nicotine component is present in a concentration from about 0.1% w/w to about 3% by weight, such as, e.g., from about 0.1% w/w to about 2.5%, from about 0.1% to about 2.0%, from about 0.1% to about 1.5%, or from about 0.1% to about 1% by weight, calculated as the free base and based on the total weight of the nanoemulsion. These ranges can also apply to other active ingredients noted herein.

In some embodiments, the nanoemulsion of the disclosure can be characterized as completely free or substantially free of nicotine. For example, certain embodiments can be characterized as having less than 0.1% by weight, or less than 0.01% by weight, or less than 0.001% by weight of a nicotine component, or 0% by weight of a nicotine component.

In some embodiments, the active ingredient is lipophilic (i.e., having significantly greater solubility in an oil phase versus an aqueous phase). Without wishing to be bound by theory, formulation of a lipophilic active ingredient as a nanoemulsion may enhance the stability of the active ingredient (e.g., toward oxidation). Nanoemulsions have small-sized droplets having greater surface area, potentially enhancing oral or mucosal absorption of the active ingredient, may assist in solubilizing the active ingredient, and/or may be helpful in masking the taste of the active ingredient.

In certain embodiments, the active ingredient is selected from the group consisting of caffeine, taurine, GABA, theanine, vitamin C, lemon balm extract, ginseng, citicoline, sunflower lecithin, and combinations thereof. For example, the active ingredient can include a combination of caffeine, theanine, and optionally ginseng. In another embodiment, the active ingredient includes a combination of theanine, gamma-amino butyric acid (GABA), and lemon balm extract. In a further embodiment, the active ingredient includes theanine, theanine and tryptophan, or theanine and one or more B vitamins (e.g., vitamin B6 or B12). In a still further embodiment, the active ingredient includes a combination of caffeine, taurine, and vitamin C.

### Flavorant

In some embodiments, the nanoemulsion comprises a flavorant. As used herein, a "flavorant" or "flavoring agent" is any flavorful or aromatic substance capable of altering the sensory characteristics associated with the nanoemulsion and/or with an oral product incorporating such a nanoemulsion. Examples of sensory characteristics that can be modified by the flavoring agent include taste, mouthfeel, moistness, coolness/heat, and/or fragrance/aroma. Flavoring agents may be natural or synthetic, and the character of the flavors imparted thereby may be described, without limitation, as fresh, sweet, herbal, confectionary, floral, fruity, or spicy. Specific types of flavors include, but are not limited to, vanilla, coffee, chocolate/cocoa, cream, mint, spearmint, menthol, peppermint, wintergreen, eucalyptus, lavender, cardamom, nutmeg, cinnamon, clove, cascarilla, sandalwood, honey, jasmine, ginger, anise, sage, licorice, lemon, orange, apple, peach, lime, cherry, strawberry, trigeminal sensates, terpenes, and any combinations thereof. See also, Leffingwell et al., Tobacco Flavoring for Smoking Products, R. J. Reynolds Tobacco Company (1972). Flavorings also may include components that are considered moistening, cooling or smoothening agents, such as eucalyptus. These flavors may be provided neat (i.e., alone) or in a composite, and may be employed as concentrates or flavor packages (e.g., spearmint and menthol, orange and cinnamon; lime, pineapple, and the like). Representative types of components also are set forth in US Pat. No. 5,387,416 to White et al.; US Pat. App. Pub. No. 2005/0244521 to Strickland et al.; and PCT Application Pub. No. WO 05/041699 to Quinter et al.. In some instances, the flavoring agent may be provided in a spray-dried form or a liquid form.

In some embodiments, the flavorant is lipophilic. Without wishing to be bound by theory, formulation of a lipophilic flavorant as a nanoemulsion may enhance the stability of the flavorant (e.g., toward oxidation or evaporation). In some embodiments, the flavorant is susceptible to oxidation, meaning exposure to air results in the degradation of components in the flavorant due to chemical changes. Examples of functional groups which may be present in flavorant components exhibiting susceptibility to oxidation include, but are not limited to, alkenes, aldehydes, and/or ketones. In some embodiments, the flavorant comprises a citrus oil. Citrus oils contain, for example, terpene components which may be susceptible to oxidation, evaporation, or both and, thus, may particularly benefit from inclusion within a product in the form of a nanoemulsion as provided herein.

The amount of flavorant utilized in the nanoemulsion can vary, but is typically up to about 10 weight percent, and certain embodiments are characterized by a flavoring agent content of at least about 0.1 weight percent, such as about 0.5 to about 10 weight percent, about 1 to about 6 weight percent, or about 2 to about 5 weight percent, based on the total weight of the nanoemulsion.

### Further components

In some embodiments, the nanoemulsion as disclosed herein may further comprise additional components, and/or the nanoemulsion may be combined with additional components to form a composition configured for oral use. The additional components may comprise one or more buffering agents, colorants, salts, sweeteners, fillers, binders, humectants, tobacco material, oral care additives, other additives, or a combination thereof. Each of these additional components is further described herein below.

### Salts

In some embodiments, the nanoemulsion or the composition comprising the nanoemulsion according to the disclosure comprises a salt (e.g., an alkali metal salt), typically employed in an amount sufficient to provide desired sensory attributes to the composition. Non-limiting examples of suitable salts include sodium chloride, potassium chloride, ammonium chloride, flour salt, sodium acetate, sodium citrate, and the like. When present, a representative amount of salt is about 0.5 percent by weight or more, about 1.0 percent by weight or more, or about 1.5 percent by weight or more, but will typically make up about 10 percent or less, or about 7.5 percent or less, or about 5 percent or less (e.g., from about 0.5 to about 5 percent by weight) of the total weight of the nanoemulsion or the composition comprising the nanoemulsion.

### Sweeteners

In order to improve the sensory properties of the nanoemulsion or the composition comprising the nanoemulsion according to the disclosure, one or more sweeteners may be added. The sweeteners can be any sweetener or combination of sweeteners, in natural or artificial form, or as a combination of natural and artificial sweeteners. Examples of natural sweeteners include fructose, sucrose, glucose, maltose, isomaltulose, mannose, galactose, lactose, stevia, honey, and the like. Examples of artificial sweeteners include sucralose, maltodextrin, saccharin, aspartame, acesulfame K, neotame and the like. In some embodiments, the sweetener comprises one or more sugar alcohols. Sugar alcohols are polyols derived from monosaccharides or disaccharides that have a partially or fully hydrogenated form. Sugar alcohols have, for example, about 4 to about 20 carbon atoms and include erythritol, arabitol, ribitol, isomalt, maltitol, dulcitol, iditol, mannitol, xylitol, lactitol, sorbitol, and combinations thereof (e.g., hydrogenated starch hydrolysates).

When present, a sweetener or combination of sweeteners may make up from about 0.1 to about 20 percent or more by weight of the of the nanoemulsion or the composition comprising the nanoemulsion, for example, from about 0.1 to about 1%, from about 1 to about 5%, from about 5 to about 10%, or from about 10 to about 20% by weight, based on the total weight of the nanoemulsion or the composition comprising the nanoemulsion.

### Buffering agents

In certain embodiments, the nanoemulsion or the composition comprising the nanoemulsion of the present disclosure can comprise pH adjusters or buffering agents. Examples of pH adjusters and buffering agents that can be used include, but are not limited to, metal hydroxides (e.g., alkali metal hydroxides such as sodium hydroxide and potassium hydroxide), and other alkali metal buffers such as metal carbonates (e.g., potassium carbonate or sodium carbonate), or metal bicarbonates such as sodium bicarbonate, and the like. Where present, the buffering agent is typically present in an amount less than about 5 percent based on the weight of the nanoemulsion or the composition comprising the nanoemulsion, for example, from about 0.5% to about 5%, such as, *e.g.,* from about 0.75% to about 4%, from about 0.75% to about 3%, or from about 1% to about 2% by weight, based on the total weight of the nanoemulsion or the composition comprising the nanoemulsion. Non-limiting examples of suitable buffers include alkali metals acetates, glycinates, phosphates, glycerophosphates, citrates, carbonates, hydrogen carbonates, borates, or mixtures thereof.

### Colorants

A colorant may be employed in amounts sufficient to provide the desired physical attributes to the nanoemulsion or the composition comprising the nanoemulsion according to the present disclosure. Examples of colorants include various dyes and pigments, such as caramel coloring and titanium dioxide. The amount of colorant utilized in the nanoemulsion or the composition comprising the nanoemulsion can vary, but when present is typically up to about 3 weight percent, such as from about 0.1%, about 0.5%, or about 1%, to about 3% by weight, based on the total weight of the nanoemulsion or the composition comprising the nanoemulsion.

### Oral care ingredients

Oral care ingredients provide the ability to inhibit tooth decay or loss, inhibit gum disease, relieve mouth pain, whiten teeth, or otherwise inhibit tooth staining, elicit salivary stimulation, inhibit breath malodor, freshen breath, or the like. For example, effective amounts of ingredients such as thyme oil, eucalyptus oil and zinc (e.g., such as the ingredients of formulations commercially available as ZYTEX^{®} from Discus Dental) can be incorporated into the nanoemulsion or composition comprising the nanoemulsion as disclosed herein. Other examples of ingredients that can be incorporated in desired effective amounts within the present nanoemulsion or the composition comprising the nanoemulsion can include those that are incorporated within the types of oral care compositions set forth in Takahashi et al., Oral Microbiology and Immunology, 19(1), 61-64 (2004); U.S. Pat. No. 6,083,527 to Thistle; and US Pat. Appl. Pub. Nos. 2006/0210488 to Jakubowski and 2006/02228308 to Cummins et al. Other exemplary ingredients include those contained in formulations marketed as MALTISORB^{®} by Roquette and DENTIZYME^{®} by NatraRx. When present, a representative amount of oral care additive is at least about 1 percent, often at least about 3 percent, and frequently at least about 5 percent of the total weight of the nanoemulsion or the composition comprising the nanoemulsion. The amount of oral care additive will not typically exceed about 30 percent, often will not exceed about 25 percent, and frequently will not exceed about 20 percent, of the total weight of the nanoemulsion or the composition comprising the nanoemulsion.

### Fillers

Compositions comprising the nanoemulsion as described herein may include a filler. The nanoemulsion as disclosed herein may be associated with a filler in various ways (i.e., in a composition comprising a nanoemulsion as disclosed herein). For example, the nanoemulsion may be disposed on the surface of a filler, may be dispersed in or impregnated into (e.g., adsorbed or absorbed) a filler, or a filler and the nanoemulsion may be present in an oral product without being physically combined or in physical contact (e.g., they may be provided separately and independently within the same product). Fillers may fulfill multiple functions, such as enhancing certain organoleptic properties such as texture and mouthfeel, enhancing cohesiveness or compressibility of the product, and the like, depending on the product and the association between the filler and the nanoemulsion. Generally, the filler is a porous particulate material and is cellulose-based. For example, fillers are any non-tobacco plant material or derivative thereof, including cellulose materials derived from such sources. Examples of cellulosic non-tobacco plant material include cereal grains (e.g., maize, oat, barley, rye, buckwheat, and the like), sugar beet (e.g., FIBREX^{®} brand filler available from International Fiber Corporation), bran fiber, and mixtures thereof. Non-limiting examples of derivatives of non-tobacco plant material include starches (e.g., from potato, wheat, rice, corn), natural cellulose, and modified cellulosic materials. Additional examples of potential fillers include maltodextrin, dextrose, calcium carbonate, calcium phosphate, lactose, mannitol, xylitol, and sorbitol. Combinations of fillers can also be used.

"Starch" as used herein may refer to pure starch from any source, modified starch, or starch derivatives. Starch is present, typically in granular form, in almost all green plants and in various types of plant tissues and organs (e.g., seeds, leaves, rhizomes, roots, tubers, shoots, fruits, grains, and stems). Starch can vary in composition, as well as in granular shape and size. Often, starch from different sources has different chemical and physical characteristics. A specific starch can be selected for inclusion in the composition based on the ability of the starch material to impart a specific organoleptic property to composition. Starches derived from various sources can be used. For example, major sources of starch include cereal grains (e.g., rice, wheat, and maize) and root vegetables (e.g., potatoes and cassava). Other examples of sources of starch include acorns, arrowroot, arracacha, bananas, barley, beans (e.g., favas, lentils, mung beans, peas, chickpeas), breadfruit, buckwheat, canna, chestnuts, colacasia, katakuri, kudzu, malanga, millet, oats, oca, Polynesian arrowroot, sago, sorghum, sweet potato, quinoa, rye, tapioca, taro, tobacco, water chestnuts, and yams. Certain starches are modified starches. A modified starch has undergone one or more structural modifications, often designed to alter its high heat properties. Some starches have been developed by genetic modifications, and are considered to be "genetically modified" starches. Other starches are obtained and subsequently modified by chemical, enzymatic, or physical means. For example, modified starches can be starches that have been subjected to chemical reactions, such as esterification, etherification, oxidation, depolymerization (thinning) by acid catalysis or oxidation in the presence of base, bleaching, transglycosylation and depolymerization (e.g., dextrinization in the presence of a catalyst), cross-linking, acetylation, hydroxypropylation, and/or partial hydrolysis. Enzymatic treatment includes subjecting native starches to enzyme isolates or concentrates, microbial enzymes, and/or enzymes native to plant materials, e.g., amylase present in corn kernels to modify corn starch.

Other starches are modified by heat treatments, such as pregelatinization, dextrinization, and/or cold water swelling processes. Certain modified starches include monostarch phosphate, distarch glycerol, distarch phosphate esterified with sodium trimetaphosphate, phosphate distarch phosphate, acetylated distarch phosphate, starch acetate esterified with acetic anhydride, starch acetate esterified with vinyl acetate, acetylated distarch adipate, acetylated distarch glycerol, hydroxypropyl starch, hydroxypropyl distarch glycerol, and starch sodium octenyl succinate.

In some embodiments, the filler is a cellulose material or cellulose derivative. One particularly suitable filler for use in the compositions described herein is microcrystalline cellulose ("MCC"). The MCC may be synthetic or semi-synthetic, or it may be obtained entirely from natural celluloses. The MCC may be selected from the group consisting of AVICEL^{®} grades PH-100, PH-102, PH-103, PH-105, PH-112, PH-113, PH-200, PH-300, PH-302, VIVACEL^{®} grades 101, 102, 12, 20 and EMOCEL^{®} grades 50M and 90M, and the like, and mixtures thereof. In one embodiment, the composition comprises MCC as the filler.

The amount of filler can vary, but when present, is typically up to about 75 percent by weight of the composition comprising the nanoemulsion, based on the total weight of the composition. A typical range of filler (e.g., MCC) within the composition can be from about 10 to about 75 percent by total weight of the composition, for example, from about 10, about 15, about 20, about 25, or about 30, to about 35, about 40, about 45, or about 50 weight percent (e.g., about 20 to about 50 weight percent or about 25 to about 45 weight percent). In certain embodiments, the amount of filler is at least about 10 percent by weight, such as at least about 20 percent, or at least about 25 percent, or at least about 30 percent, or at least about 35 percent, or at least about 40 percent, based on the total weight of the composition.

### Binders

A binder (or combination of binders) may be employed in certain embodiments, in amounts sufficient to provide the desired physical attributes and physical integrity to the composition, and binders also often function as thickening or gelling agents. Typical binders can be organic or inorganic, or a combination thereof. Representative binders include cellulose derivatives (e.g., cellulose ethers), povidone, sodium alginate, starch-based binders, pectin, gums, carrageenan, pullulan, zein, and the like, and combinations thereof. In some embodiments, the binder comprises pectin or carrageenan or combinations thereof.

The amount of binder utilized in the composition can vary, but is typically up to about 30 weight percent, and certain embodiments are characterized by a binder content of at least about 0.1% by weight, such as about 1 to about 30% by weight, or about 5 to about 10% by weight, based on the total weight of the composition.

In one embodiment, the binder comprises a cellulose derivative. In certain embodiments, the cellulose derivative is a cellulose ether (including carboxyalkyl ethers), meaning a cellulose polymer with the hydrogen of one or more hydroxyl groups in the cellulose structure replaced with an alkyl, hydroxyalkyl, or aryl group. Non-limiting examples of such cellulose derivatives include methylcellulose, hydroxypropylcellulose ("HPC"), hydroxypropylmethylcellulose ("HPMC"), hydroxyethyl cellulose, and carboxymethylcellulose ("CMC"). In one embodiment, the cellulose derivative is one or more of methylcellulose, HPC, HPMC, hydroxyethyl cellulose, and CMC. In one embodiment, the cellulose derivative is HPC. In one embodiment, the cellulose derivative is a combination of HPC and HPMC. In some embodiments, the composition comprises from about 1 to about 10% of the cellulose derivative by weight, based on the total weight of the composition, with certain embodiments comprising about 1 to about 5% by weight of cellulose derivative, for example, from about 1%, about 2%, or about 3%, to about 4%, or about 5% by weight of the composition.

In certain embodiments, the binder includes a gum, for example, a natural gum. As used herein, a natural gum refers to polysaccharide materials of natural origin that have binding properties, and which are also useful as a thickening or gelling agents. Representative natural gums derived from plants, which are typically water soluble to some degree, include xanthan gum, guar gum, gum arabic, ghatti gum, gum tragacanth, karaya gum, locust bean gum, gellan gum, and combinations thereof. When present, natural gum binder materials are typically present in an amount of up to about 5% by weight, for example, from about 0.1, about 0.2, about 0.3, about 0.4, about 0.5, about 0.6, about 0.7, about 0.8, about 0.9, or about 1%, to about 2, about 3, about 4, or about 5% by weight, based on the total weight of the composition.

### Humectants

In certain embodiments, one or more humectants may be employed in the composition comprising the nanoemulsion of the present disclosure. Examples of humectants include, but are not limited to, glycerin, propylene glycol, and the like. Where included, the humectant is typically provided in an amount sufficient to provide desired moisture attributes to the composition. Further, in some instances, the humectant may impart desirable flow characteristics to the composition for depositing in a mold. When present, a humectant will typically make up about 5% or less of the weight of the composition (e.g., from about 0.5 to about 5% by weight). When present, a representative amount of humectant is about 0.1% to about 1% by weight, or about 1% to about 5% by weight, based on the total weight of the composition.

### Tobacco material

In some embodiments, the nanoemulsion or the composition comprising the nanoemulsion of the present disclosure may include a tobacco material. The tobacco material can vary in species, type, and form. Generally, the tobacco material is obtained from for a harvested plant of the *Nicotiana* species. Example *Nicotiana* species include N. tabacum, N. rustica, N. alata, N. arentsii, N. excelsior, N. forgetiana, N. glauca, N. glutinosa, N. gossei, N. kawakamii, N. knightiana, N. langsdorffi, N. otophora, N. setchelli, N. sylvestris, N. tomentosa, N. tomentosiformis, N. undulata, N. x sanderae, N. africana, N. amplexicaulis, N. benavidesii, N. bonariensis, N. debneyi, N. longiflora, N. maritina, N. megalosiphon, N. occidentalis, N. paniculata, N. plumbaginifolia, N. raimondii, N. rosulata, N. simulans, N. stocktonii, N. suaveolens, N. umbratica, N. velutina, N. wigandioides, N. acaulis, N. acuminata, N. attenuata, N. benthamiana, N. cavicola, N. clevelandii, N. cordifolia, N. corymbosa, N. fragrans, N. goodspeedii, N. linearis, N. miersii, N. nudicaulis, N. obtusifolia, N. occidentalis subsp. Hersperis, N. pauciflora, N. petunioides, N. quadrivalvis, N. repanda, N. rotundifolia, N. solanifolia, and N. spegazzinii. Various representative other types of plants from the *Nicotiana* species are set forth in Goodspeed, The Genus Nicotiana, (Chonica Botanica) (1954); US Pat. Nos. 4,660,577 to Sensabaugh, Jr. et al.; 5,387,416 to White et al., 7,025,066 to Lawson et al.; 7,798,153 to Lawrence, Jr. and 8,186,360 to Marshall et al.. Descriptions of various types of tobaccos, growing practices and harvesting practices are set forth in Tobacco Production, Chemistry and Technology, Davis et al. (Eds.) (1999).

*Nicotiana* species from which suitable tobacco materials can be obtained can be derived using genetic-modification or crossbreeding techniques (e.g., tobacco plants can be genetically engineered or crossbred to increase or decrease production of components, characteristics or attributes). See, for example, the types of genetic modifications of plants set forth in US Pat. Nos. 5,539,093 to Fitzmaurice et al.; 5,668,295 to Wahab et al.; 5,705,624 to Fitzmaurice et al.; 5,844,119 to Weigl; 6,730,832 to Dominguez et al.; 7,173,170 to Liu et al.; 7,208,659 to Colliver et al. and 7,230,160 to Benning et al.; US Patent Appl. Pub. No. 2006/0236434 to Conkling et al.; and PCT WO2008/103935 to Nielsen et al. See, also, the types of tobaccos that are set forth in US Pat. Nos. 4,660,577 to Sensabaugh, Jr. et al.; 5,387,416 to White et al.; and 6,730,832 to Dominguez et al..

The *Nicotiana* species can, in some embodiments, be selected for the content of various compounds that are present therein. For example, plants can be selected on the basis that those plants produce relatively high quantities of one or more of the compounds desired to be isolated therefrom. In certain embodiments, plants of the *Nicotiana* species (e.g., *Galpao commun* tobacco) are specifically grown for their abundance of leaf surface compounds. Tobacco plants can be grown in greenhouses, growth chambers, or outdoors in fields, or grown hydroponically.

Various parts or portions of the plant of the *Nicotiana* species can be included within a nanoemulsion or composition as disclosed herein, as disclosed herein. For example, virtually all of the plant (*e.g.*, the whole plant) can be harvested, and employed as such. Alternatively, various parts or pieces of the plant can be harvested or separated for further use after harvest. For example, the flower, leaves, stem, stalk, roots, seeds, and various combinations thereof, can be isolated for further use or treatment. In some embodiments, the tobacco material comprises tobacco leaf (lamina). The nanoemulsion or composition as disclosed herein can include processed tobacco parts or pieces, cured and aged tobacco in essentially natural lamina and/or stem form, a tobacco extract, extracted tobacco pulp (e.g., using water as a solvent), or a mixture of the foregoing (e.g., a mixture that combines extracted tobacco pulp with granulated cured and aged natural tobacco lamina).

In certain embodiments, the tobacco material comprises solid tobacco material selected from the group consisting of lamina and stems. The tobacco that is used for the mixture most preferably includes tobacco lamina, or a tobacco lamina and stem mixture (of which at least a portion is smoke-treated). Portions of the tobaccos within the mixture may have processed forms, such as processed tobacco stems (e.g., cut-rolled stems, cut-rolled-expanded stems or cut-puffed stems), or volume expanded tobacco (e.g., puffed tobacco, such as dry ice expanded tobacco (DIET)). See, for example, the tobacco expansion processes set forth in US Pat. Nos. 4,340,073 to de la Burde et al.; 5,259,403 to Guy et al.; and 5,908,032 to Poindexter, et al.; and 7,556,047 to Poindexter, et al.. In addition, the composition optionally may incorporate tobacco that has been fermented. See, also, the types of tobacco processing techniques set forth in PCT Application Publication No. WO2005/063060 to Atchley et al..

Where used within a nanoemulsion or composition as disclosed herein, the tobacco material is typically used in a form that can be described as particulate (i.e., shredded, ground, granulated, or powder form). The tobacco plant or portion thereof can be separated into individual parts or pieces (e.g., the leaves can be removed from the stems, and/or the stems and leaves can be removed from the stalk). The harvested plant or individual parts or pieces can be further subdivided into parts or pieces (e.g., the leaves can be shredded, cut, comminuted, pulverized, milled or ground into pieces or parts that can be characterized as filler-type pieces, granules, particulates or fine powders).

The manner by which the tobacco material is provided in a finely divided or powder type of form may vary. Preferably, plant parts or pieces are comminuted, ground or pulverized into a particulate form using equipment and techniques for grinding, milling, or the like. Most preferably, the plant material is relatively dry in form during grinding or milling, using equipment such as hammer mills, cutter heads, air control mills, or the like. For example, tobacco parts or pieces may be ground or milled when the moisture content thereof is less than about 15 weight percent or less than about 5 weight percent. The plant, or parts thereof, can be subjected to external forces or pressure (e.g., by being pressed or subjected to roll treatment). When carrying out such processing conditions, the plant or portion thereof can have a moisture content that approximates its natural moisture content (e.g., its moisture content immediately upon harvest), a moisture content achieved by adding moisture to the plant or portion thereof, or a moisture content that results from the drying of the plant or portion thereof. For example, powdered, pulverized, ground or milled pieces of plants or portions thereof can have moisture contents of less than about 25 weight percent, often less than about 20 weight percent, and frequently less than about 15 weight percent. Most preferably, the tobacco material is employed in the form of parts or pieces that have an average particle size between 1.4 millimeters and 250 microns. In some instances, the tobacco particles may be sized to pass through a screen mesh to obtain the particle size range required. If desired, air classification equipment may be used to ensure that small sized tobacco particles of the desired sizes, or range of sizes, may be collected. If desired, differently sized pieces of granulated tobacco may be mixed together.

For the preparation of oral products, it is typical for a harvested plant of the *Nicotiana* species to be subjected to a curing process. The tobacco materials incorporated within the composition for inclusion within products as disclosed herein are those that have been appropriately cured and/or aged. Descriptions of various types of curing processes for various types of tobaccos are set forth in Tobacco Production, Chemistry and Technology, Davis et al. (Eds.) (1999). Examples of techniques and conditions for curing flue-cured tobacco are set forth in Nestor et al., Beitrage Tabakforsch. Int., 20, 467-475 (2003) and US Pat. No. 6,895,974 to Peele. Representative techniques and conditions for air curing tobacco are set forth in US Pat. No. 7,650,892 to Groves et al.; Roton et al., Beitrage Tabakforsch. Int., 21, 305-320 (2005) and Staaf et al., Beitrage Tabakforsch. Int., 21, 321-330 (2005). Certain types of tobaccos can be subjected to alternative types of curing processes, such as fire curing or sun curing.

In certain embodiments, tobacco materials that can be employed include flue-cured or Virginia (e.g., K326), burley, sun-cured (e.g., Indian Kurnool and Oriental tobaccos, including Katerini, Prelip, Komotini, Xanthi and Yambol tobaccos), Maryland, dark, dark-fired, dark air cured (e.g., Madole, Passanda, Cubano, Jatin and Bezuki tobaccos), light air cured (e.g., North Wisconsin and Galpao tobaccos), Indian air cured, Red Russian and *Rustica* tobaccos, as well as various other rare or specialty tobaccos and various blends of any of the foregoing tobaccos.

The tobacco material may also have a so-called "blended" form. For example, the tobacco material may include a mixture of parts or pieces of flue-cured, burley (e.g., Malawi burley tobacco) and Oriental tobaccos (e.g., as tobacco composed of, or derived from, tobacco lamina, or a mixture of tobacco lamina and tobacco stem). For example, a representative blend may incorporate about 30 to about 70 parts burley tobacco (e.g., lamina, or lamina and stem), and about 30 to about 70 parts flue cured tobacco (e.g., stem, lamina, or lamina and stem) on a dry weight basis. Other example tobacco blends incorporate about 75 parts flue-cured tobacco, about 15 parts burley tobacco, and about 10 parts Oriental tobacco; or about 65 parts flue-cured tobacco, about 25 parts burley tobacco, and about 10 parts Oriental tobacco; or about 65 parts flue-cured tobacco, about 10 parts burley tobacco, and about 25 parts Oriental tobacco; on a dry weight basis. Other example tobacco blends incorporate about 20 to about 30 parts Oriental tobacco and about 70 to about 80 parts flue-cured tobacco on a dry weight basis.

Tobacco materials used in the present disclosure can be subjected to, for example, fermentation, bleaching, and the like. If desired, the tobacco materials can be, for example, irradiated, pasteurized, or otherwise subjected to controlled heat treatment. Such treatment processes are detailed, for example, in US Pat. No. 8,061,362 to Mua et al.. In certain embodiments, tobacco materials can be treated with water and an additive capable of inhibiting reaction of asparagine to form acrylamide upon heating of the tobacco material (e.g., an additive selected from the group consisting of lysine, glycine, histidine, alanine, methionine, cysteine, glutamic acid, aspartic acid, proline, phenylalanine, valine, arginine, compositions incorporating di- and trivalent cations, asparaginase, certain non-reducing saccharides, certain reducing agents, phenolic compounds, certain compounds having at least one free thiol group or functionality, oxidizing agents, oxidation catalysts, natural plant extracts (e.g., rosemary extract), and combinations thereof). See, for example, the types of treatment processes described in US Pat. Pub. Nos. 8,434,496, 8,944,072, and 8,991,403 to Chen et al.. In certain embodiments, this type of treatment is useful where the original tobacco material is subjected to heat in the processes previously described.

In various embodiments, the tobacco material can be treated to extract a soluble component of the tobacco material therefrom. "Tobacco extract" as used herein refers to the isolated components of a tobacco material that are extracted from solid tobacco pulp by a solvent that is brought into contact with the tobacco material in an extraction process. Various extraction techniques of tobacco materials can be used to provide a tobacco extract and tobacco solid material. See, for example, the extraction processes described in US Pat. Appl. Pub. No. 2011/0247640 to Beeson et al.. Other example techniques for extracting components of tobacco are described in US Pat. Nos. 4,144,895 to Fiore; 4,150,677 to Osborne, Jr. et al.; 4,267,847 to Reid; 4,289,147 to Wildman et al.; 4,351,346 to Brummer et al.; 4,359,059 to Brummer et al.; 4,506,682 to Muller; 4,589,428 to Keritsis; 4,605,016 to Soga et al.; 4,716,911 to Poulose et al.; 4,727,889 to Niven, Jr. et al.; 4,887,618 to Bernasek et al.; 4,941,484 to Clapp et al.; 4,967,771 to Fagg et al.; 4,986,286 to Roberts et al.; 5,005,593 to Fagg et al.; 5,018,540 to Grubbs et al.; 5,060,669 to White et al.; 5,065,775 to Fagg; 5,074,319 to White et al.; 5,099,862 to White et al.; 5,121,757 to White et al.; 5,131,414 to Fagg; 5,131,415 to Munoz et al.; 5,148,819 to Fagg; 5,197,494 to Kramer; 5,230,354 to Smith et al.; 5,234,008 to Fagg; 5,243,999 to Smith; 5,301,694 to Raymond et al.; 5,318,050 to Gonzalez-Parra et al.; 5,343,879 to Teague; 5,360,022 to Newton; 5,435,325 to Clapp et al.; 5,445,169 to Brinkley et al.; 6,131,584 to Lauterbach; 6,298,859 to Kierulff et al.; 6,772,767 to Mua et al.; and 7,337,782 to Thompson.

In some embodiments, the type of tobacco material is selected such that it is initially visually lighter in color than other tobacco materials to some degree (e.g., whitened or bleached). Tobacco pulp can be whitened in certain embodiments according to any means known in the art.

Typical inclusion ranges for tobacco materials can vary depending on the nature and type of the tobacco material, and the intended effect on the nanoemulsion, composition, or final product incorporating the same, with an example range of up to about 30% by weight (or up to about 20% by weight or up to about 10% by weight or up to about 5% by weight), based on total weight of the composition (e.g., about 0.1 to about 15% by weight). In some embodiments, the products of the disclosure (nanoemulsions, nanoemulsion-containing compositions, and compositions comprising the same) can be characterized as completely free or substantially free of tobacco material (other than purified nicotine as a possible active ingredient). In some embodiments, such products are described as having no tobacco material (other than purified nicotine as a possible active ingredient) intentionally added thereto. For example, certain embodiments can be characterized as having less than 1% by weight, or less than 0.5% by weight, or less than 0.1% by weight of tobacco material, or 0% by weight of tobacco material based on the weight of the nanoemulsion, the nanoemulsion-containing composition, or the product incorporating the nanoemulsion or composition.

### Other additives

Other additives can be included in the nanoemulsion or the composition comprising the nanoemulsion as disclosed. For example, the nanoemulsion or the composition comprising the nanoemulsion can be processed, blended, formulated, combined, and/or mixed with other materials or ingredients. The additives can be artificial, or can be obtained or derived from herbal or biological sources. Examples of further types of additives include thickening or gelling agents (e.g., fish gelatin), preservatives (e.g., potassium sorbate and the like), disintegration aids, zinc or magnesium salts selected to be relatively water soluble for compositions with greater water solubility (e.g., magnesium or zinc gluconate) or selected to be relatively water insoluble for compositions with reduced water solubility (e.g., magnesium or zinc oxide), or combinations thereof. See, for example, those representative components, combination of components, relative amounts of those components, and manners and methods for employing those components, set forth in US Pat. No. 9,237,769 to Mua et al., US Pat. No. 7,861,728 to Holton, Jr. et al., US Pat. App. Pub. No. 2010/0291245 to Gao et al., and US Pat. App. Pub. No. 2007/0062549 to Holton, Jr. et al.. Typical inclusion ranges for such additional additives can vary depending on the nature and function of the additive and the intended effect on the final composition, with an example range of up to about 10% by weight, (e.g., about 0.1 to about 5% by weight) based on total weight of the nanoemulsion or the composition comprising the nanoemulsion.

The aforementioned additives can be employed together (e.g., as additive formulations) or separately (e.g., individual additive components can be added at different stages involved in the preparation of the final product). Furthermore, the aforementioned types of additives may be encapsulated as provided in the final product or composition. Exemplary encapsulated additives are described, for example, in WO2010/132444 to Atchley.

### Preparation of nanoemulsions

Nanoemulsions as disclosed herein can be prepared by mechanical processes which employ shear force to break large emulsion droplets into smaller ones, such as high-pressure homogenization (HPH, including microfluidization), high-amplitude ultrasonic processing, and ultrasound-assisted emulsification. In general, the nanoemulsions of the present disclosure can be prepared by preparing an aqueous phase containing an emulsifying agent as disclosed herein (e.g., an amphiphilic molecule or surfactant) and homogenizing this solution with a homogenizer or mixer for a period of time; and preparing an oil phase containing an oil, as described herein above. One or more hydrophobic active ingredients, flavors, or combinations thereof, as desired, may be added to the aqueous and/or oil phase, followed by mixing the same with a suitable mixing device. The aqueous and oil phases are combined and homogenized with, for example, a probe sonicator (Sonics and Materials, USA), a high pressure homogenizer (such as one made by Gauline or Avestine, or the like), or a microfluidizer, to obtain the desired nanoemulsion. The number of passes through a high pressure homogenizer/microfluidizer may vary, depending on the desired particle size for the nanoemulsions. A variety of methods are known in the art for producing nanoemulsions comprising nano-sized particles of particular size ranges, using for example, sonication or homogenization. One such method is described in U.S. Pat. No. 4,737,323.

### Nanoemulsion properties

Nanoemulsions as disclosed herein generally comprise nano-scale particles having an average size of from 20 to 200 nm, optionally from about 20 to about 100 nm, or optionally from about 40 to about 100 nm. In some embodiments, the average particle size is about 100, about 90, about 80, about 70, about 60, about 50 or about 40 nm. In some embodiments, the average particle size is from about 40 to about 60 nm. In some embodiments, the average particle size is from about 40 to about 60 nm, and the nanoemulsion is transparent.

The size of the nanoparticles may be determined by quasi-electric light scattering (QELS) as described in Bloomfield, Ann. Rev. Biophys. Bioeng., 10:421-450 (1981). It may also be measured by correlation spectroscopy that analyzes the fluctuation in scattering of light due to Brownian motion, or by transmission electron microscopy (TEM).

The nanoemulsion as disclosed herein may be characterized by reference to a polydispersity index. Polydispersity indicates the uniformity of droplet size in a nanoemulsion. The higher the value of polydispersity, the lower will be the uniformity of droplet size. It may be defined as the ratio of standard deviation to mean droplet size. It may be measured by spectrophotometric methods. In some embodiments, it may be advantageous to provide nanoemulsions with a low polydispersity index, e.g., less than about 0.5. In some embodiments, the nanoemulsion has a polydispersity index of less than about 0.3.

The nanoemulsion as disclosed herein may be characterized by reference to zeta potential. Zeta potential is a measure of the charge on the surface of droplet in nanoemulsion. In some embodiments, the zeta potential of the nanoparticles is from about -40 mV to about 40 mV.

### Configured for oral use

The nanoemulsion and compositions and products comprising the nanoemulsion as described herein are configured for oral use. The term "configured for oral use" as used herein means that the product is provided in a form such that during use, saliva in the mouth of the user causes one or more of the components of the nanoemulsion, composition, or product (e.g., flavoring agents and/or active ingredients) to pass into the mouth of the user. In certain embodiments, the nanoemulsion, composition, or product is adapted to deliver components to a user through mucous membranes in the user's mouth, the user's digestive system, or both, and, in some instances, said component is an active ingredient that can be absorbed through the mucous membranes in the mouth or absorbed through the digestive tract when the product is used.

Products configured for oral use (into which the disclosed nanoemulsion or composition are incorporated) may take various forms, including gels, pastilles, gums, lozenges, powders, and pouches. The product configured for oral use according to the present invention is in the form of a pouched product. Gels can be soft or hard. Certain products configured for oral use are in the form of pastilles. As used herein, the term "pastille" refers to a dissolvable oral product made by solidifying a liquid or gel composition so that the final product is a somewhat hardened solid gel. The rigidity of the gel is highly variable. Certain products of the disclosure are in the form of solids. Certain products can exhibit, for example, one or more of the following characteristics: crispy, granular, chewy, syrupy, pasty, fluffy, smooth, and/or creamy. In certain embodiments, the desired textural property can be selected from the group consisting of adhesiveness, cohesiveness, density, dryness, fracturability, graininess, gumminess, hardness, heaviness, moisture absorption, moisture release, mouthcoating, roughness, slipperiness, smoothness, viscosity, wetness, and combinations thereof.

The products comprising the nanoemulsions or compositions of the present disclosure may be dissolvable. As used herein, the terms "dissolve," "dissolving," and "dissolvable" refer to compositions having aqueous-soluble components that interact with moisture in the oral cavity and enter into solution, thereby causing gradual consumption of the product. According to one aspect, the dissolvable product is capable of lasting in the user's mouth for a given period of time until it completely dissolves. Dissolution rates can vary over a wide range, from about 1 minute or less to about 60 minutes. For example, fast release compositions typically dissolve and/or release the active substance in about 2 minutes or less, often about 1 minute or less (e.g., about 50 seconds or less, about 40 seconds or less, about 30 seconds or less, or about 20 seconds or less). Dissolution can occur by any means, such as melting, mechanical disruption (e.g., chewing), enzymatic or other chemical degradation, or by disruption of the interaction between the components of the composition. In some embodiments, the product can be meltable as discussed, for example, in US Patent App. Pub. No. 2012/0037175 to Cantrell et al. In other embodiments, the products do not dissolve during the product's residence in the user's mouth.

In one embodiment, the product of the present disclosure is in the form of a composition comprising the nanoemulsion as described herein, disposed within a moisture-permeable container (e.g., a water-permeable pouch). Such compositions in the water-permeable pouch format are typically used by placing one pouch containing the composition in the mouth of a human subject/user. Generally, the pouch is placed somewhere in the oral cavity of the user, for example under the lips, in the same way as moist snuff products are generally used. The pouch preferably is not chewed or swallowed. Exposure to saliva then causes some of the components of the composition therein (e.g., flavoring agents and/or active ingredients) to pass through e.g., the water-permeable pouch and provide the user with flavor and satisfaction, and the user is not required to spit out any portion of the composition. After about 10 minutes to about 60 minutes, typically about 15 minutes to about 45 minutes, of use/enjoyment, substantial amounts of the composition absorbed through oral mucosa of the human subject, and the pouch may be removed from the mouth of the human subject for disposal.

The nanoemulsion or composition as disclosed herein and any other components noted above are combined within a moisture-permeable packet or pouch that acts as a container for use of the composition to provide a pouched product configured for oral use. Certain embodiments of the disclosure will be described with reference to Fig. 1 of the accompanying drawing, and these described embodiments involve snus-type products having an outer pouch and containing a composition as described herein. As explained in greater detail below, such embodiments are provided by way of example only, and the pouched products of the present disclosure can include the composition in other forms. The composition/construction of such packets or pouches, such as the container pouch **102** in the embodiment illustrated in Fig. 1, may be varied. Referring to Fig. 1, there is shown a first embodiment of a pouched product **100.** The pouched product **100** includes a moisture-permeable container in the form of a pouch **102**, which contains a material **104** comprising a nanoemulsion or nanoemulsion-containing composition as described herein.

Suitable packets, pouches or containers of the type used for the manufacture of smokeless tobacco products are available under the tradenames CatchDry, Ettan, General, Granit, Goteborgs Rape, Grovsnus White, Metropol Kaktus, Mocca Anis, Mocca Mint, Mocca Wintergreen, Kicks, Probe, Prince, Skruf and TreAnkrare. The composition may be contained in pouches and packaged, in a manner and using the types of components used for the manufacture of conventional snus types of products. The pouch provides a liquid-permeable container of a type that may be considered to be similar in character to the mesh-like type of material that is used for the construction of a tea bag. Components of the composition readily diffuse through the pouch and into the mouth of the user.

Non-limiting examples of suitable types of pouches are set forth in, for example, US Pat. Nos. 5,167,244 to Kjerstad and 8,931,493 to Sebastian et al.; as well as US Patent App. Pub. Nos. 2016/0000140 to Sebastian et al.; 2016/0073689 to Sebastian et al.; 2016/0157515 to Chapman et al.; and 2016/0192703 to Sebastian et al.. Pouches can be provided as individual pouches, or a plurality of pouches (e.g., 2, 4, 5, 10, 12, 15, 20, 25 or 30 pouches) can be connected or linked together (e.g., in an end-to-end manner) such that a single pouch or individual portion can be readily removed for use from a one-piece strand or matrix of pouches.

An example pouch may be manufactured from materials, and in such a manner, such that during use by the user, the pouch undergoes a controlled dispersion or dissolution. Such pouch materials may have the form of a mesh, screen, perforated paper, permeable fabric, or the like. For example, pouch material manufactured from a mesh-like form of rice paper, or perforated rice paper, may dissolve in the mouth of the user. As a result, the pouch and composition each may undergo complete dispersion within the mouth of the user during normal conditions of use, and hence the pouch and composition both may be ingested by the user. Other examples of pouch materials may be manufactured using water dispersible film forming materials (e.g., binding agents such as alginates, carboxymethylcellulose, xanthan gum, pullulan, and the like), as well as those materials in combination with materials such as ground cellulosics (e.g., fine particle size wood pulp). Preferred pouch materials, though water dispersible or dissolvable, may be designed and manufactured such that under conditions of normal use, a significant amount of the composition contents permeate through the pouch material prior to the time that the pouch undergoes loss of its physical integrity. If desired, flavoring ingredients, disintegration aids, and other desired components, may be incorporated within, or applied to, the pouch material.

The amount of nanoemulsion or composition contained within each product unit, for example, a pouch, may vary. In some embodiments, the weight of the nanoemulsion or composition within each pouch is at least about 50 mg, for example, from about 50 mg to about 2 grams, from about 100 mg to about 1.5 grams, or from about 200 to about 700 mg. In some smaller embodiments, the weight of the nanoemulsion or composition within each pouch may be from about 100 to about 300 mg. For a larger embodiment, the weight of the material within each pouch may be from about 300 mg to about 700 mg. If desired, other components can be contained within each pouch. For example, at least one flavored strip, piece or sheet of flavored water dispersible or water soluble material (e.g., a breath-freshening edible film type of material) may be disposed within each pouch along with or without at least one capsule. Such strips or sheets may be folded or crumpled in order to be readily incorporated within the pouch. See, for example, the types of materials and technologies set forth in US Pat. Nos. 6,887,307 to Scott et al. and 6,923,981 to Leung et al.; and The EFSA Journal (2004) 85, 1-32.

In certain embodiments, one or more active ingredients as described herein are included in the composition within the pouched product, and one or more further active ingredients are disposed in or on the external surface of the product (e.g., on or in the pouch material as disclosed herein). In some embodiments, separate location of the active ingredients may allow differential release profiles (e.g., one active ingredient may be rapidly available to the mouth and/or digestive system, and the other active ingredient may be released more gradually with product use).

A pouched product as described herein can be packaged within any suitable inner packaging material and/or outer container. See also, for example, the various types of containers for smokeless types of products that are set forth in US Pat. Nos. 7,014,039 to Henson et al.; 7,537,110 to Kutsch et al.; 7,584,843 to Kutsch et al.; 8,397,945 to Gelardi et al., D592,956 to Thiellier; D594,154 to Patel et al.; and D625,178 to Bailey et al.; US Pat. Pub. Nos. 2008/0173317 to Robinson et al.; 2009/0014343 to Clark et al.; 2009/0014450 to Bjorkholm; 2009/0250360 to Bellamah et al.; 2009/0266837 to Gelardi et al.; 2009/0223989 to Gelardi; 2009/0230003 to Thiellier; 2010/0084424 to Gelardi; and 2010/0133140 to Bailey et al; 2010/0264157 to Bailey et al.; and 2011/0168712 to Bailey et al..

Many modifications and other embodiments of the invention will come to mind to one skilled in the art to which this invention pertains having the benefit of the teachings presented in the foregoing description. Therefore, it is to be understood that the invention is not to be limited to the specific embodiments disclosed.

Although specific terms are employed herein, they are used in a generic and descriptive sense only and not for purposes of limitation.

## Claims

1. A pouched product configured for oral use, comprising a filler and a nanoemulsion dispersed in or disposed on the filler, the nanoemulsion comprising nanoparticles having a size from 20 to 200 nm, the nanoemulsion comprising:
a vegetable or mineral oil;
water;
from 0.01% to 15% by weight of an emulsifying agent wherein the emulsifying agent is a surfactant, a phospholipid, an amphiphilic polysaccharide, an amphiphilic protein, or a combination thereof; and
an active ingredient selected from the group consisting of botanical materials, stimulants, amino acids, vitamins, antioxidants, nicotine components, cannabinoids, cannabimimetics, terpenes, nutraceuticals, pharmaceutical agents, and combinations thereof, the nanoemulsion optionally further comprising a stabilizer selected from the group consisting of polysaccharides and polyols.

2. The pouched product of claim 1, wherein the oil is a vegetable oil comprising a long chain fatty acid, a monoacylglycerol, a diacylglycerol, a triacylglycerol, or a combination thereof, wherein the acyl group is a long chain fatty acid.

3. The pouched product of claim 1, wherein the oil comprises mineral oil, or comprises castor oil, corn oil, coconut oil, evening primrose oil, linseed oil, peanut oil, soybean oil, safflower oil, flaxseed oil, sunflower oil, olive oil, or a combination thereof.

4. The pouched product of any one of claims 1-3, wherein the active ingredient is lipophilic.

5. The pouched product of any one of claims 1-4, wherein the emulsifying agent comprises Tween 20, Tween 80, Span 20, Span 40, Span 60, Span 80, lecithin, a hydrocolloid gum, a modified starch, or a combination thereof.

6. The pouched product of any one of claims 1-5, wherein one or more of the following applies:
the zeta potential of the nanoparticles is from -40 mV to 40 mV; and/or
the nanoemulsion comprises particles having a polydispersity index of less than 0.3.

7. The pouched product of any one of claims 1-6, comprising less than 0.01% by weight of nicotine.

8. The pouched product of any one of claims 1-7, wherein the active ingredient is selected from the group consisting of caffeine, taurine, GABA, theanine, vitamin C, lemon balm extract, ginseng, citicoline, sunflower lecithin, and combinations thereof.

9. The pouched product of claim 8, wherein the active ingredient is selected from the group consisting of caffeine, theanine, and optionally ginseng.

10. The pouched product of claim 8, wherein the active ingredient is selected from the group consisting of theanine, gamma-amino butyric acid (GABA), and lemon balm extract.

11. The pouched product of claim 8, wherein the active ingredient is selected from the group consisting of theanine, and optionally tryptophan or one or more B vitamins.

12. The pouched product of claim 8, wherein the active ingredient is selected from the group consisting of caffeine, taurine, and vitamin C.

13. The pouched product of any one of claims 1-12, further comprising:
a flavourant; or
an active ingredient disposed in or on an external surface of the pouched product.

14. The pouched product of claim 13, wherein the flavorant is lipophilic, optionally, wherein the flavorant comprises a citrus oil.

## Patentansprüche

1. Beutelprodukt, das zur oralen Verwendung konfiguriert ist, das einen Füllstoff und eine Nanoemulsion umfasst, die in dem Füllstoff dispergiert oder auf diesem angeordnet ist, wobei die Nanoemulsion Nanopartikel mit einer Größe von 20 bis 200 nm umfasst, wobei die Nanoemulsion Folgendes umfasst:
ein Pflanzen- oder Mineralöl;
Wasser;
von 0,01 bis 15 Gew.-% eines Emulgators, wobei der Emulgator ein Tensid, ein Phospholipid, ein amphiphiles Polysaccharid, ein amphiphiles Protein oder eine Kombination davon ist; und
einen Wirkstoff, der aus der Gruppe ausgewählt ist, die aus botanischen Materialien, Stimulanzien, Aminosäuren, Vitaminen, Antioxidantien, Nikotinkomponenten, Cannabinoiden, Cannabimimetika, Terpenen, Nutrazeutika, pharmazeutischen Mitteln und Kombinationen davon besteht, wobei die Nanoemulsion optional ferner einen Stabilisator umfasst, der aus der Gruppe ausgewählt ist, die aus Polysacchariden und Polyolen besteht.

2. Beutelprodukt nach Anspruch 1, wobei das Öl ein Pflanzenöl ist, das eine langkettige Fettsäure, ein Monoacylglycerol, ein Diacylglycerol, ein Triacylglycerol oder eine Kombination davon umfasst, wobei die Acylgruppe eine langkettige Fettsäure ist.

3. Beutelprodukt nach Anspruch 1, wobei das Öl Mineralöl umfasst oder Rizinusöl, Maisöl, Kokosnussöl, Nachtkerzenöl, Leinsamenöl, Erdnussöl, Sojaöl, Distelöl, Flachssamenöl, Sonnenblumenöl, Olivenöl oder eine Kombination davon umfasst.

4. Beutelprodukt nach einem der Ansprüche 1-3, wobei der Wirkstoff lipophil ist.

5. Beutelprodukt nach einem der Ansprüche 1-4, wobei der Emulgator Tween 20, Tween 80, Span 20, Span 40, Span 60, Span 80, Lecithin, ein Hydrokolloidgummi, eine modifizierte Stärke oder eine Kombination davon umfasst.

6. Beutelprodukt nach einem der Ansprüche 1-5, wobei eines oder mehrere von Folgendem zutrifft/zutreffen:
das Zetapotential der Nanopartikel beträgt von -40 mV bis 40 mV; und/oder
die Nanoemulsion umfasst Partikel mit einem Polydispersitätsindex von weniger als 0,3.

7. Beutelprodukt nach einem der Ansprüche 1-6, weniger als 0,01 Gew.-% Nikotin umfassend.

8. Beutelprodukt nach einem der Ansprüche 1-7, wobei der Wirkstoff aus der Gruppe ausgewählt ist, die aus Koffein, Taurin, GABA, Theanin, Vitamin C, Zitronenmelissenextrakt, Ginseng, Citicolin, Sonnenblumenlecithin und Kombinationen davon besteht.

9. Beutelprodukt nach Anspruch 8, wobei der Wirkstoff aus der Gruppe ausgewählt ist, die aus Koffein, Theanin und optional Ginseng besteht.

10. Beutelprodukt nach Anspruch 8, wobei der Wirkstoff aus der Gruppe ausgewählt ist, die aus Theanin, Gamma-Aminobuttersäure (GABA) und Zitronenmelissenextrakt besteht.

11. Beutelprodukt nach Anspruch 8, wobei der Wirkstoff aus der Gruppe ausgewählt ist, die aus Theanin und optional Tryptophan oder einem oder mehreren B-Vitaminen besteht.

12. Beutelprodukt nach Anspruch 8, wobei der Wirkstoff aus der Gruppe ausgewählt ist, die aus Koffein, Taurin und Vitamin C besteht.

13. Beutelprodukt nach einem der Ansprüche 1-12, ferner Folgendes umfassend:
einen Geschmacksstoff; oder
einen Wirkstoff, der in oder auf einer äußeren Oberfläche des Beutelprodukts angeordnet ist.

14. Beutelprodukt nach Anspruch 13, wobei der Geschmacksstoff lipophil ist, wobei der Geschmacksstoff optional ein Zitrusöl umfasst.

## Revendications

1. Produit en pochette conçu pour un usage oral, comprenant une charge et une nanoémulsion dispersée dans ou disposée sur la charge, la nanoémulsion comprenant des nanoparticules ayant une taille de 20 à 200 nm, la nanoémulsion comprenant :
une huile végétale ou minérale ;
de l'eau ;
de 0,01 % à 15 % en poids d'un agent émulsifiant, dans lequel l'agent émulsifiant est un tensioactif, un phospholipide, un polysaccharide amphiphile, une protéine amphiphile ou une combinaison de ceux-ci ; et
un ingrédient actif choisi dans le groupe constitué de matières botaniques, stimulants, acides aminés, vitamines, antioxydants, composants de nicotine, cannabinoïdes, cannabimimétiques, terpènes, produits nutraceutiques, agents pharmaceutiques et des combinaisons de ceux-ci, la nanoémulsion comprenant éventuellement en outre un stabilisant choisi dans le groupe constitué de polysaccharides et de polyols.

2. Produit en pochette selon la revendication 1, dans lequel l'huile est une huile végétale comprenant un acide gras à longue chaîne, un monoacylglycérol, un diacylglycérol, un triacylglycérol ou une combinaison de ceux-ci, dans lequel le groupe acyle est un acide gras à longue chaîne.

3. Produit en pochette selon la revendication 1, dans lequel l'huile comprend de l'huile minérale, ou comprend de l'huile de ricin, huile de maïs, huile de coco, huile d'onagre, huile de lin, huile d'arachide, huile de soja, huile de carthame, huile de lin, huile de tournesol, huile d'olive, ou une combinaison de celles-ci.

4. Produit en pochette selon l'une quelconque des revendications 1 à 3, dans lequel l'ingrédient actif est lipophile.

5. Produit en pochette selon l'une quelconque des revendications 1 à 4, dans lequel l'agent émulsifiant comprend du Tween 20, Tween 80, Span 20, Span 40, Span 60, Span 80, lécithine, une gomme hydrocolloïde, un amidon modifié ou une combinaison de ceux-ci.

6. Produit en pochette selon l'une quelconque des revendications 1 à 5, dans lequel une ou plusieurs des caractéristiques suivantes s'appliquent :
le potentiel zêta des nanoparticules est de -40 mV à 40 mV ; et/ou
la nanoémulsion comprend des particules ayant un indice de polydispersité inférieur à 0,3.

7. Produit en pochette selon l'une quelconque des revendications 1 à 6, comprenant moins de 0,01 % en poids de nicotine.

8. Produit en pochette selon l'une quelconque des revendications 1 à 7, dans lequel l'ingrédient actif est choisi dans le groupe constitué par la caféine, taurine, GABA, théanine, vitamine C, extrait de mélisse, ginseng, citicoline, lécithine de tournesol et des combinaisons de ceux-ci.

9. Produit en pochette selon la revendication 8, dans lequel l'ingrédient actif est choisi dans le groupe constitué par la caféine, théanine et éventuellement ginseng.

10. Produit en pochette selon la revendication 8, dans lequel l'ingrédient actif est choisi dans le groupe constitué par la théanine, l'acide gamma-amino butyrique (GABA) et l'extrait de mélisse.

11. Produit en pochette selon la revendication 8, dans lequel l'ingrédient actif est choisi dans le groupe constitué par la théanine et éventuellement le tryptophane ou une ou plusieurs vitamines B.

12. Produit en pochette selon la revendication 8, dans lequel l'ingrédient actif est choisi dans le groupe constitué par la caféine, la taurine et la vitamine C.

13. Produit en pochette selon l'une quelconque des revendications 1 à 12, comprenant en outre :
un arôme ; ou
un ingrédient actif disposé dans ou sur une surface externe du produit en pochette.

14. Produit en pochette selon la revendication 13, dans lequel l'arôme est lipophile, éventuellement, dans lequel l'arôme comprend une huile d'agrumes.
